# EUROPEAN PATENT SPECIFICATION

(11) **EP 0 904 747 B1**
(45) Date of publication and mention of the grant of the patent: **08.12.2004**
(21) Application number: 98116270.4
(22) Date of filing: 28.08.1998
(51) Int. Cl.: A61F 2/16

(54) **Intraocular lens**
Intraokularlinse
Lentille intraoculaire

(30) Priority: 29.08.1997 JP 24939597
(43) Date of publication of application: 31.03.1999
(73) Proprietor: Nidek Co., Ltd., Gamagori-shi, Aichi (JP)
(72) Inventor: Sunada, Tsutomu, Toyohashi-shi, Aichi (JP)
(74) Representative: Weber, Joachim, Dr.

(56) References cited:
- WO-A-96/35397
- FR-A- 2 661 816
- US-A- 4 676 791
- US-A- 5 002 568
- US-A- 5 002 571

## Description

The present invention relates to an intraocular lens to be inserted into the eye in place of the crystalline lens extirpated due to cataract, etc. Document WO 96/35397A discloses the preamble of claim 1.

### BACKGROUND OF THE INVENTION

Known as one of eye diseases is cataract, in which the crystalline lens of the eye clouds gradually. Techniques of cataract treatment include an intraocular-lens insertion operation in which the clouded crystalline lens is extirpated from the eye and an intraocular lens is inserted.

Although the intraocular-lens insertion operation is generally conducted by a method comprising inserting an intraocular lens into the crystalline-lens capsule from which the crystalline lens has been removed by extirpation, this operation sometimes leads to secondary cataract, which is a postoperative complication in which the posterior capsule becomes cloudy. This secondary cataract is thought to be a clouding of the posterior capsule which occurs when crystalline-lens epithelial cells, which are present mainly in the anterior capsule, proliferate and invade the posterior capsule. The clouding of the posterior capsule is not limited to one point but extends throughout the whole posterior capsule due to proliferation. The symptoms of secondary cataract therefore are the same as those of cataract.

Various investigations have been made in order to prevent secondary cataract, and it has been reported that use of a lens whose surface has tackiness (stickiness) is effective in inhibiting secondary cataract. This inhibition is achieved by the following mechanism. Since the lens has tackiness (stickiness), adhesion occurs in the contact areas between the lens surface and each of the posterior capsule and the anterior capsule. This adhesion substantially inhibits crystalline-lens epithelial cells, which are the main cause of secondary cataract, from reaching the back of the lens. As a result, the lens is kept clear on the back side and throughout its surface.

However, this conventional tacky (sticky) lens has a problem of poor handleability although use thereof is effective in inhibiting secondary cataract. Namely, because of its tackiness (stickiness), the lens tends to stick to an insertion instrument used for inserting the lens into the eye and be unable to be separated therefrom. In the case of a foldable intraocular lens, the folded lens tends to adhere to itself and not to open.

US-A-5 002 571 describes an intraocular lens for implanting in the posterior chamber of a human eye after an extracapsular extraction, the intraocular lens having adhesive means applied to its backside thereof. The intraocular lens is manufactured of a hard material which is not foldable and, therefore, is a so-called hard lens.

FR-A-2 661 816 discloses an intraocular lens having a margin which is uneven or which comprises through-holes or slots so that the lens can be fixed to the anterior capsule and the posterior capsule with a fibrose.

It is an object of the present invention to provide a foldable intraocular lens according to the preamble portion of claim 1 which has satisfactory handleability while taking advantage of a tacky (sticky) lens surface which functions to diminish secondary cataract.

According to the invention, the object is solved by the features of the independent claims. The respective sub-claims contain further preferred developments of the invention.

### BRIEF DESCRIPTION OF THE DRAWINGS

Fig. 1 is a view illustrating the shape of an intraocular lens.
Figs. 2A and 2B are views illustrating one example of a process for producing the optical part of the intraocular lens as an embodiment of the present invention.
Figs. 3A and 3B are views illustrating one example of a process for integrally forming the optical part and the supporting parts of the intraocular lens.
Figs. 4A-4C are views illustrating the movement of the optical part during the insertion of the intraocular lens into the eye.

### DETAILED DESCRIPTION OF THE INVENTION

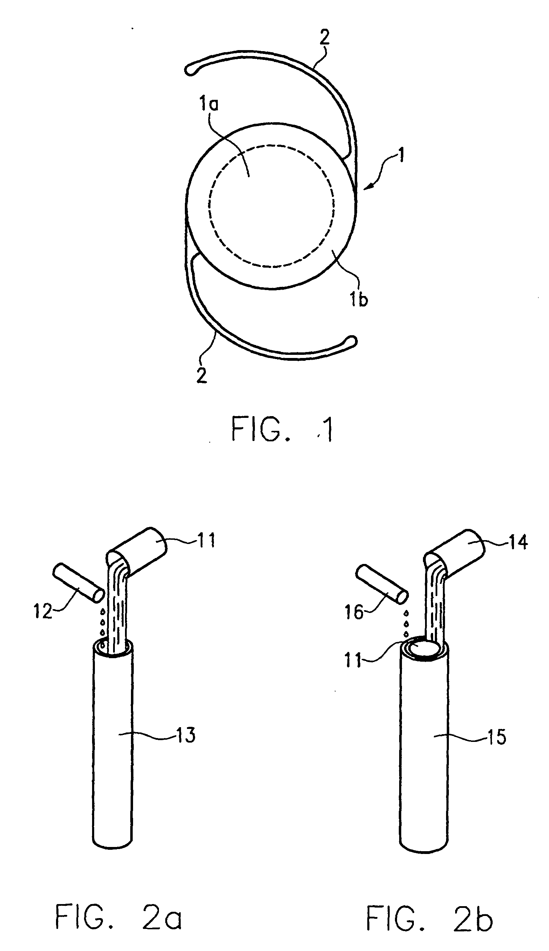

An embodiment of the present invention is explained below by reference to the drawings. Fig. 1 is a plan view illustrating a one-piece type intraocular lens according to the present invention. Numeral 1 denotes an optical part having refracting power. The optical part 1 comprises a central part 1a constituted of a nontacky (nonsticky) material and a peripheral part 1b constituted of a tacky (sticky) material (these materials will be described later). The range of the tacky (sticky) peripheral part 1b is concentrically distributed around the center of the optical part 1. Each numeral 2 denotes a supporting part which serves to keep the optical part 1 within the eye, and has flexibility.

An example of a process for producing this intraocular lens is then explained. First, as shown in Fig. 2A, a liquid optical member 11 for forming the central part 1a made of a nontacky (nonsticky) material is poured into a cylindrical reaction vessel 13. The size of the reaction vessel 13 used is about two-thirds the diameter of the optical part 1 of the intraocular lens. However, the size thereof is not limited thereto and varies depending on the degree of tackiness (stickiness) and the material properties of the peripheral part 1b. Usable example of the material of the nontacky (nonsticky) central part 1a include polymers of hydrous or anhydrous, acrylic and vinyl compounds such as methyl methacrylate, ethyl methacrylate, hydroxyethyl methacrylate, ethylhexyl methacrylate, and polyvinylpyrrolidone. A crosslinking agent represented by ethylene glycol dimethacrylate (EGDMA) may be added to these materials.

Subsequently, a polymerization initiator 12 serving to initiate polymerization is added in a small amount to the optical member 11 which has been poured into the reaction vessel 13. Examples of the polymerization initiator 12 include many conventionally known polymerization initiators. For example, azobisisobutyronitrile, azobisdimethylvaleronitrile, or the like can be used for heat polymerization, while benzoin, methyl o-benzoylbenzoate, or the like can be used for photopolymerization.

After addition of the polymerization initiator 12, the contents are heated (or irradiated with light) to initiate polymerization. As a result, the polymerization initiator 12 generates radicals (free radicals) due to the heat (or light). When the polymerization initiator 12 which has generated radicals collides with molecules of the optical member 11, the polymerization initiator 12 combines with the optical member 11. The radicals which have been present in the polymerization initiator 12 disappear after serving as a linkage factor for the optical member 11, and this results in the generation of radicals in the optical member 11. Thereafter, radical-possessing molecules of the optical member 11 collide and combine with other molecules of the optical member 11, and this collision/combination occurs repeatedly. Thus, radical generation and polymerization/curing proceed as a chain reaction.

This polymerization is conducted for a sufficiently long period, whereby the remaining radicals which have generated combine with one another to terminate the polymerization reaction. As a result, the optical member 11 comes into a completely polymerized and cured state in which no radicals are present.

Thereafter, the thus-obtained completely polymerized and cured, cylindrical shape of the optical member 11 is placed in a cylindrical reaction vessel 15 and fixed thereto nearly at the center thereof as shown in Fig. 2B. The reaction vessel 15 used has a diameter not smaller than that of the optical part 1 of the intraocular lens. After the optical member 11 has been fixed, an optical member 14 which becomes the tacky (sticky) peripheral part 1b is poured into the space surrounding the optical member 11. Usable examples of the material of the optical member 14 include acrylates such as ethyl acrylate, propyl acrylate, butyl acrylate, dodecyl acrylate, 2-hydroxypropyl acrylate, and phenyl ethyl acrylate.

After the optical member 14 has been poured, a polymerization initiator 16 is introduced and heat (light) etc. is then applied to the contents to polymerize and cure the same, as in the case of the optical member 11. For this polymerization, either the same polymerization initiator as that used for curing the optical member 11 or a different one may be used.

Thus, a composite member 17 consisting of the optical member 11 and the optical member 14 is produced. Subsequently, a composite member consisting of the composite member 17 and a supporting member 18 is produced in the same manner as the above.

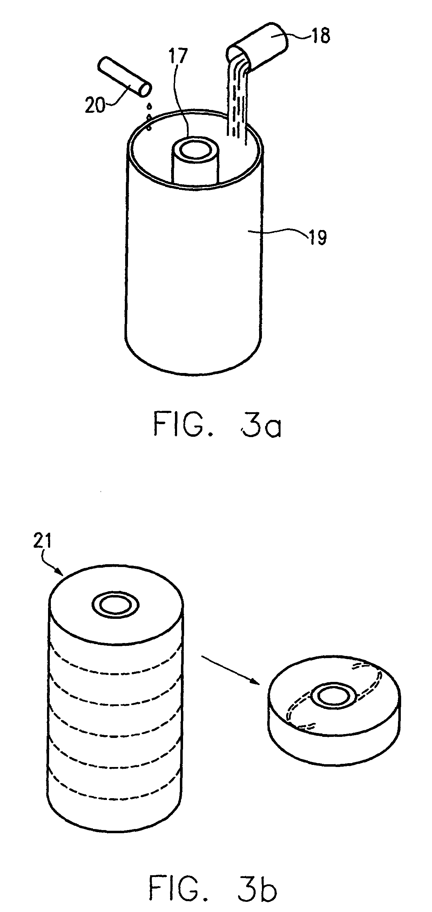

After the completely polymerized and solidified, cylindrical composite member 17 consisting of the optical members 11 and 14 has been obtained, this composite member 17 is placed in a cylindrical reaction vessel 19 and fixed thereto nearly at the center thereof as shown in Fig. 3A. The reaction vessel 19 has a diameter not smaller than the whole length of the intraocular lens. After the composite member 17 has been fixed, a supporting member 18 which becomes the supporting parts 2 is poured into the space surrounding the composite member 17. Acrylic esters and methacrylic esters can be used as the supporting member 20. Examples of the material of the supporting parts 2 include a copolymer obtained by copolymerizing a monomer which gives a flexible polymer, e.g., 2-ethylhexyl methacrylate, with a rigid material represented by PMMA, EMA, etc.

After the supporting member 18 has been poured, a polymerization initiator 20 is introduced into the reaction vessel 19 and heat (light) etc. is applied to the contents to polymerize and cure the same. For this polymerization, either the same polymerization initiator as that used for curing the optical member 11 or 14 or a different one may be used.

Thus, a composite member 21 consisting of the composite member 17 and the supporting member 18 is obtained. Thereafter, this composite member 21 is taken out of the reaction vessel 19 and cut into a necessary thickness as shown in Fig. 3B. Each cut piece is then subjected to a known cutting work to thereby obtain an intraocular lens having an optical part 1 and supporting parts 2.

Although the optical member 11 serving as a nontacky (nonsticky) part was polymerized and cured first in the process described above, it is possible to polymerize and cure the optical member 14 and the supporting member 18 before the optical member 11 is polymerized. Furthermore, in the process described above, one material was completely polymerized and cured, before the next material was poured to produce a composite material. However, it is possible to employ a process in which the material poured first into a reaction vessel is polymerized and cured to some degree, and the next step is initiated before the first material completely polymerizes and cures, i.e., at the time when the first material is in such a state that many radicals are still present therein. This process gives an intraocular lens in which the bonding areas have a higher bonding strength than those of the embodiment described above, because molecules of one material combine with molecules of another.

A tacky (sticky) material can be obtained also by copolymerizing two or more different materials. However, all the materials to be copolymerized need not be monomers which each gives a tacky (sticky) polymer, as long as the copolymerization results in a composite material having such tackiness (stickiness) that the posterior capsule can adhere thereto. Consequently, there are many possible combinations with respect to material selection, comonomer material proportion, etc.

The optical part 1, which has a two-layer structure consisting of the nontacky (nonsticky) central part 1a and the tacky (sticky) peripheral part 1b, may produce an influence due to a difference in refractive index between the two layers. In this case, the two layers can be easily made to have the same refractive index by suitably incorporating an additive for changing refractive index or by copolymerizing a material having a high refractive index with a material having a low refractive index to control the refractive index of the resultant copolymer.

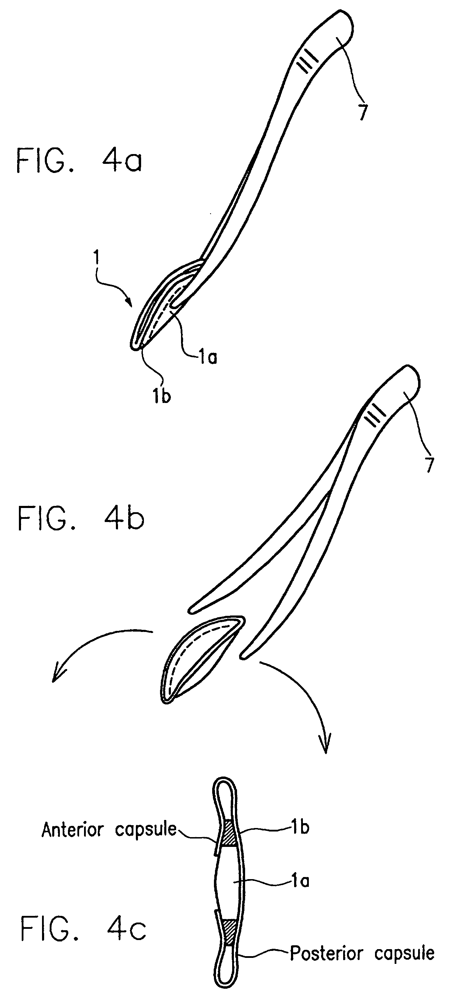

An example of insertion into the eye is explained next with respect to an intraocular lens obtained in the manner described above. The intraocular lens used here as an example is a foldable intraocular lens in which the optical part 1 is constituted of a central part 1a and a peripheral part 1b each made of a flexible material. The insertion of this intraocular lens is explained by reference to Figs. 4A-4C (the supporting parts 2 are not shown in the figures).

First, the cloudy crystalline lens is broken into pieces, emulsified, and removed, for example, by the known technique based on ultrasonic emulsification and suction. Thereafter, as in Fig. 4A the optical part 1 is bent by nipping the central part 1a, made of a nontacky (nonsticky) material, between the tips of an insertion instrument (forceps) 7, and inserted into the crystalline-lens capsule from which the nucleus of lens has been removed. After the insertion, the intraocular lens is positioned within the eye and the arms of the insertion instrument 7 are then opened slowly. Since the nipped part of the optical part 1 is the nontacky (nonsticky) part (central part 1a), the insertion instrument 7 has been weakly adherent to the lends surface and is hence more easily separated therefrom than from a lens in which the whole surface is made of a tacky (sticky) material. When the arms of the insertion instrument 7 are opened, the restoring force of the intraocular lens allows the lens to return to its original shape as shown in Fig. 4B. In this stage also, since the optical part 1 has a nontacky (nonsticky) surface part (central part 1a), the optical part 1 has a small area where the part 1 can adhere to itself and is hence more apt to return to its original shape than an intraocular lens in which the whole optical part 1 is constituted of a tacky (sticky) material.

After the optical part 1 has returned to its original shape, a surface thereof is brought into contact with the posterior capsule as shown in Fig. 4C. Since the tacky (sticky) peripheral part 1b adheres to the posterior capsule within the crystalline-lens capsule, crystalline-lens epithelial cells which have proliferated can be sufficiently prevented from reaching inside beyond the peripheral part 1b. Thus, secondary cataract can be prevented.

Furthermore, since the tacky (sticky) part has been formed concentrically around the center of the optical part 1, the central part of the lens can be kept clear, which is important for ensuring postoperative vision.

Although the embodiment described above had a constitution in which a nontacky (nonsticky) material and a tacky (sticky) material were used as the central part 1a and the peripheral part 1b, respectively, an intraocular lens having the reverse constitution is possible. In this case, the central part 1a adheres to the posterior capsule within the crystalline-lens capsule, in contrast with the above embodiment. However, by ensuring an area of the adhesion thereof, crystalline-lens epithelial cells which have proliferated can be prevented from reaching the central part on the posterior capsule side.

The proportion of the tacky (sticky) surface to the nontacky (nonsticky) surface is suitably determined based on a balance between the arrangement of these surfaces and the respective functions thereof. However, in the case where the peripheral part is tacky (sticky), the proportion of the surface thereof is preferably from 15% to about 55%. In the case where the central part is tacky (sticky), the proportion of the surface thereof is preferably from 25% to about 55%.

Although the embodiment described above was a one-piece foldable soft lens, as a typical intraocular lens, the present invention is applicable to any kind of intraocular lens as long as the optical part thereof employs a tacky (sticky) material. For example, the present invention is applicable, without posing a problem, to a three-piece lens.

As described above, according to the present invention, both the inhibition of secondary cataract and good handleability during lens insertion can be attained due to the tacky (sticky) lens surface. Furthermore, in the case of a foldable lens, the restoration of shape is easy.

## Claims

1. A foldable intraocular lens to be inserted into a crystalline-lens capsule after crystalline-lens extirpation, which comprises an optical part (1) having a predetermined refractive power,
**characterized in that**
the optical part is divided into a central part (1a) and a peripheral part (1b), and **in that** at least one of the central and peripheral parts is made of a tacky material so that one of the central and peripheral parts has a tacky. surface and the other has a nontacky surface.

2. The intraocular lens according to claim 1, **characterized in that** the central part of the optical part is made of the tacky material and the peripheral part of the optical part is made of a nontacky material so that the central part has the tacky surface and the peripheral part has the nontacky surface.

3. The intraocular lens according to claim 2, wherein the proportion of the tacky surface on one side is from 25 to 55%.

4. The intraocular lens according to claim 1, **characterized in that** the peripheral part of the optical part is made of the tacky material and the central part of the optical part is made of a nontacky material so that the peripheral part has the tacky surface and the central part has the nontacky surface.

5. The intraocular lens according to claim 4, wherein the proportion of the tacky surface on one side is from 15 to 55%.

6. The intraocular lens according to any one of claims 1 to 5, wherein the tacky material is formed from at least one acrylate selected from the group consisting of ethyl acrylate, propyl acrylate, butyl acrylate, dodecyl acrylate, 2-hydroxypropyl acrylate, and phenyl ethyl acrylate.

7. The intraocular lens according to any one of claims 2 to 5, wherein the nontacky material is formed from at least one acrylic compound selected from the group consisting of methyl methacrylate, ethyl methacrylate, hydroxyethyl methacrylate, and ethylhexyl methacrylate.

8. A method of producing a foldable intraocular lens to be inserted into a crystalline-lens capsule after crystalline-lens extirpation, **characterized in that** the method comprises the steps of:
- pouring a liquid tacky material (11) into a first cylindrical vessel (13);
- polymerizing and curing the liquid tacky material;
- placing the polymerized and cured tacky material substantially at the center of a second cylindrical vessel (15) having a larger diameter than the first cylindrical vessel;
- pouring a liquid nontacky material (14) into the second cylindrical vessel; and
- polymerizing and curing the liquid nontacky material,
- whereby an optical part (1) of the intraocular lens having a predetermined refractive power and including a tacky central part (1a) and a nontacky peripheral part (1b) is produced.

9. A method of producing a foldable intraocular lens to be inserted into a crystalline-lens capsule after crystalline-lens extirpation, **characterized in that** the method comprises the steps of:
- pouring a liquid nontacky material (11) into a first cylindrical vessel (13);
- polymerizing and curing the liquid nontacky material;
- placing the polymerized and cured nontacky material substantially at the center of a second cylindrical vessel (15) having a larger diameter than the first cylindrical vessel;
- pouring a liquid tacky material (14) into the second cylindrical vessel; and
- polymerizing and curing the liquid tacky material,
- whereby an optical part (1) of the intraocular lens having a predetermined refractive power and including a nontacky central part (1a) and a tacky peripheral part (1b) is produced.

10. The method of producing the intraocular lens according to claim 8 or 9, **characterized by** the steps of:
- placing the polymerized and cured tacky material and nontacky material constituting the optical part (1) substantially at the center of a third cylindrical vessel (19) having a larger diameter than the second cylindrical vessel;
- pouring a liquid material (18) for a supporting part (2) for holding the optical part in the crystalline-lens capsule into the third cylindrical vessel; and
- polymerizing and curing the liquid material for the support part,
- whereby the intraocular lens including the optical part (1) and the support part (2) is produced.

## Patentansprüche

1. Faltbare intraokulare Linse zum Einsetzen in eine Kristalllinsenkapsel nach Entfernung der Kristalllinse, welche einen optischen Teil (1) mit einer vorbestimmten Refraktionsstärke umfasst, **dadurch gekennzeichnet, dass** der optische Teil in einen Mittelteil (1a) und einen Umfangsteil (1b) unterteilt ist, und dass zumindest das Mittelteil und/oder das Umfangsteil aus klebendem Material besteht, so dass ein Teil, Mittelteil oder Umfangsteil, eine klebende Oberfläche und das andere Teil eine nicht-klebende Oberfläche aufweist.

2. Intraokulare Linse nach Anspruch 1, **dadurch gekennzeichnet, dass** der Mittelteil des optischen Teils aus einem klebenden Material besteht und der Umfangsteil des optischen Teils aus einem nicht-klebenden Material besteht, so dass der Mittelteil eine klebende Oberfläche und der Umfangsteil eine nicht-klebende Oberfläche aufweist.

3. Intraokulare Linse nach Anspruch 2, wobei der Anteil der klebenden Oberfläche auf einer Seite zwischen 25 bis 55 % beträgt.

4. Intraokulare Linse nach Anspruch 1, **dadurch gekennzeichnet, dass** der Umfangsteil des optischen Teils aus einem klebenden Material besteht und der Mittelteil des optischen Teils aus einem nicht-klebenden Material besteht, so dass der Umfangsteil eine klebende Oberfläche und der Mittelteil eine nicht-klebende Oberfläche aufweist.

5. Intraokulare Linse nach Anspruch 4, wobei der Anteil der klebenden Oberfläche auf einer Seite zwischen 15 und 55 % beträgt.

6. Intraokulare Linse nach einem der Ansprüche 1 bis 5, wobei das klebende Material aus zumindest einem Acrylat ausgewählt aus der Gruppe bestehend aus Ethylacrylat, Propylacrylat, Butylacrylat, Dodecylacrylat, 2-Hydroxy-Propylacrylat und Phenylethylacrylat, gebildet ist.

7. Intraokulare Linse nach einem der Ansprüche 2 bis 5, wobei das nicht-klebende Material aus zumindest einem Acrylbestandteil ausgewählt aus der Gruppe bestehend aus Methylmethacrylat, Ethylmethacrylat, Hydraxyethyl-Methacrylat und Ethylhexyl-Methacrylat, gebildet ist.

8. Verfahren zur Herstellung einer faltbaren intraokularen Linse zum Einsetzen in eine Kristalllinsenkapsel nach Entfernung der Kristalllinse, **dadurch gekennzeichnet, dass** das Verfahren die Schritte umfasst:
- Gießen eines flüssigen klebenden Materials (11) in ein erstes zylindrisches Gefäß (13);
- Polymerisieren und Härten des flüssigen klebenden Materials;
- Plazieren des polymerisierten und gehärteten klebenden Materials im Wesentlichen in der Mitte eines zweiten zylindrischen Gefäßes (15) mit einem größeren Durchmesser als das erste zylindrische Gefäß;
- Gießen eines flüssigen nicht-klebenden Materials (14) in das zweite zylindrische Gefäß; und
- Polymerisieren und Härten des flüssigen nicht-klebenden Materials,
- wobei ein optischer Teil (1) der intraokularen Linse mit einer vorbestimmten Refraktionsstärke und mit einem klebenden Mittelteil (1a) und einem nicht-klebenden Umfangsteil (1b) hergestellt wird.

9. Verfahren zur Herstellung einer faltbaren intraokularen Linse zum Einsetzen in eine Kristalllinsenkapsel nach Entfernung der Kristalllinse, **dadurch gekennzeichnet, dass** das Verfahren die Schritte umfasst:
- Gießen eines flüssigen nicht-klebenden Materials (11) in ein erstes zylindrisches Gefäß (13);
- Polymerisieren und Härten des flüssigen nicht-klebenden Materials;
- Plazieren des polymerisierten und gehärteten nicht-klebenden Materials im Wesentlichen in der Mitte eines zweiten zylindrischen Gefäßes (15) mit einem größeren Durchmesser als das erste zylindrische Gefäß;
- Gießen eines flüssigen klebenden Materials (14) in das zweite zylindrische Gefäß; und
- Polymerisieren und Härten des flüssigen klebenden Materials,
- wobei ein optischer Teil (1) der intraokularen Linse mit einer vorbestimmten Refraktionsstärke und mit einem nicht-klebenden Mittelteil (1a) und einem klebenden Umfangsteil (1b) hergestellt wird.

10. Verfahren zur Herstellung der intraokularen Linse nach Anspruch 8 oder 9, **gekennzeichnet durch** die Schritte:
- Plazieren des polymerisierten und gehärteten klebenden Materials und nicht-klebenden Materials, welche den optischen Teil (1) bilden, im Wesentlichen in der Mitte eines dritten zylindrischen Gefäßes (19) mit einem größeren Durchmesser als das zweite zylindrische Gefäß;
- Gießen eines flüssigen Materials (18) für ein Stützteil (2) zum Halten des optischen Teils in der Kristalllinsenkapsel in das dritte zylindrische Gefäß; und
- Polymerisieren und Härten des flüssigen Materials für das Stützteil,
- wobei die intraokulare Linse mit dem optischen Teil (1) und dem Stützteil (2) hergestellt wird.

## Revendications

1. Lentille intraoculaire pliable à insérer dans une capsule de cristallin après extirpation du cristallin, qui comprend une partie optique (1) ayant une puissance optique prédéterminée,
**caractérisée en ce que**
la partie optique est divisée en une partie centrale (1a) et une partie périphérique (1b), et **en ce qu'**au moins une des parties centrale et périphérique est composée d'un matériau collant de manière à ce qu'une des parties centrale et périphérique ait une surface collante et que l'autre ait une surface non collante.

2. Lentille intraoculaire selon la revendication 1, **caractérisée en ce que** la partie centrale de la partie optique est composée du matériau collant et que la partie périphérique de la partie optique est composée d'un matériau non collant de manière à ce que la partie centrale ait la surface collante et que la partie périphérique ait la surface non collante.

3. Lentille intraoculaire selon la revendication 2, dans laquelle la proportion de la surface collante sur un côté est comprise entre 25 et 55 %.

4. Lentille intraoculaire selon la revendication 1, **caractérisée en ce que** la partie périphérique de la partie optique est composée du matériau collant et la partie centrale de la partie optique est composée d'un matériau non collant de manière à ce que la partie périphérique ait la surface collante et la partie centrale ait la surface non collante.

5. Lentille intraoculaire selon la revendication 4, dans laquelle la proportion de la surface collante sur un côté est comprise entre 15 et 55 %.

6. Lentille intraoculaire selon l'une quelconque des revendications 1 à 5, dans laquelle le matériau collant est formé à partir d'au moins un acrylate choisi dans le groupe constitué par l'acrylate d'éthyle, l'acrylate de propyle, l'acrylate de butyle, l'acrylate de dodécyle, l'acrylate de 2-hydroxypropyle et l'acrylate de phényléthyle.

7. Lentille intraoculaire selon l'une quelconque des revendications 2 à 5, dans laquelle le matériau non collant est formé à partir d'au moins un composé acrylique choisi dans le groupe constitué par le méthacrylate de méthyle, le méthacrylate d'éthyle, le méthacrylate d'hydroxyéthyle et le méthacrylate d'éthylhexyle.

8. Procédé de production d'une lentille intraoculaire pliable à insérer dans une capsule de cristallin après extirpation du cristallin, **caractérisé en ce que** le procédé comprend les étapes consistant à :
- verser un matériau collant liquide (11) dans une première cuve cylindrique (13) ;
- polymériser et durcir le matériau collant liquide ;
- placer le matériau collant polymérisé et durci substantiellement au centre d'une deuxième cuve cylindrique (15) ayant un diamètre supérieur à celui de la première cuve cylindrique ;
- verser un matériau non collant liquide (14) dans la deuxième cuve cylindrique ; et
- polymériser et durcir le matériau non collant liquide,
- moyennant quoi une partie optique (1) de la lentille intraoculaire ayant une puissance optique prédéterminée et comprenant une partie centrale collante (1a) et une partie périphérique non collante (1b) est produite.

9. Procédé de production d'une lentille intraoculaire pliable à insérer dans une capsule de cristallin après extirpation du cristallin, **caractérisé en ce que** le procédé comprend les étapes consistant à :
- verser un matériau non collant liquide (11) dans une première cuve cylindrique (13) ;
- polymériser et durcir le matériau non collant liquide ;
- placer le matériau non collant polymérisé et durci substantiellement au centre d'une deuxième cuve cylindrique (15) ayant un diamètre supérieur à celui de la première cuve cylindrique ;
- verser un matériau collant liquide (14) dans la deuxième cuve cylindrique; et
- polymériser et durcir le matériau collant liquide,
- moyennant quoi une partie optique (1) de la lentille intraoculaire ayant une puissance optique prédéterminée et comprenant une partie centrale non collante (1a) et une partie périphérique collante (1b) est produite.

10. Procédé de production de la lentille intraoculaire selon la revendication 8 ou 9, **caractérisé par** les étapes consistant à :
- placer le matériau collant polymérisé et durci et le matériau non collant constituant la partie optique (1) substantiellement au centre d'une troisième cuve cylindrique (19) ayant un diamètre supérieur à celui de la deuxième cuve cylindrique ;
- verser un matériau liquide (18) pour une partie de support (2) pour maintenir la partie optique dans la capsule de cristallin dans la troisième cuve cylindrique ; et
- polymériser et durcir le matériau liquide pour la partie de support,
- moyennant quoi la lentille intraoculaire comprenant la partie optique (1) et la partie de support (2) est produite.
